# EUROPÄISCHE PATENTSCHRIFT

(11) **EP 0 866 067 B1**
(45) Veröffentlichungstag und Bekanntmachung des Hinweises auf die Patenterteilung: **07.10.1998**
(21) Anmeldenummer: 97109714.2
(22) Anmeldetag: 14.06.1997
(51) Int. Cl.: C07D 451/06

(54) **Verfahren zum Herstellen von Endo-Tropin**
Process of preparation of endo-tropine
Procédé pour la préparation de l'endotropine

(43) Veröffentlichungstag der Anmeldung: 23.09.1998
(73) Patentinhaber: Dr. R. Pfleger Chemische Fabrik GmbH, 96052 Bamberg (DE)
(72) Erfinder: Sachse, Rolf, 13465 Berlin (DE); Schaupp, Albert, 96129 Strullendorf (DE)
(74) Vertreter: Fleck, Thomas, Dr. Dipl.-Chem.

(56) Entgegenhaltungen:
- US-A- 2 366 760
- US-A- 2 746 976
- CHEMICAL ABSTRACTS, vol. 99, no. 15, 10.Oktober 1983 Columbus, Ohio, US; abstract no. 122721a, KOLLAR LASZLO ET AL.: "Stereoselective reduction of nortropinones with a homogeneous catalyst" Seite 676; Spalte 1; XP002041183 & MAGY. KEM. LAPJA, Bd. 38, Nr. 4-5, 1983, Seiten 223-225,
- CHEMICAL ABSTRACTS, vol. 93, no. 13, 29.September 1980 Columbus, Ohio, US; abstract no. 132665a, MAKSUDOV, A. M. ET AL.: "Tropine preparation method" Seite 683; Spalte 2; XP002041184 & UZB. KHIM. ZH., Nr. 2, 1980, Seiten 30-33,
- CHEMICAL ABSTRACTS, vol. 97, no. 23, 6.Dezember 1982 Columbus, Ohio, US; abstract no. 198428q, CHOU, TA SHUE: "Stereoselective preparation of Tropine" Seite 600; Spalte 2; XP002041185 & K'O HSUEH FA CHAN YUEH K'AN, Bd. 10, Nr. 8, 1982, Seiten 699-702,
- CHEMICAL ABSTRACTS, vol. 83, no. 23, 8.Dezember 1975 Columbus, Ohio, US; abstract no. 193561d, JAIN, M. P. ET AL.: "Modified process for the synthesis of tropine." Seite 493; Spalte 1; XP002041186 & RES. IND., Bd. 20, Nr. 1, 1975, Seiten 3-5,

## Beschreibung

Die Erfindung betrifft ein Verfahren zum Herstellen von endo-Tropin durch Reduktion von Tropanon mit katalytisch durch Raney-Nickel- oder Raney-Kupfer-Katalysator erregtem Wasserstoff. Andere gebräuchliche Bezeichnungen für Tropin sind: 3α-Tropanol, 3α-Hydroxytropan, 1αH, 5αH-Tropan-3α-ol, 8-Methyl-8-azabicyclo-[3.2.1]nonan-3α -ol (CAS [120-29-6]).

endo-Tropin ist ein wichtiger Stoff zur Herstellung von Nortropin durch oxidative Methylierung, das seinerseits zur Herstellung der wichtigen Azoniaspironortropanolester dient, die als Pharmazeutica, insbesondere Spasmolytika, eingesetzt werden (vgl. hierzu die DE-PS 1 194 42).

Der Stand der Technik stellt sich wie folgt dar: Die US-PS 2,366,760 von 1945 beschreibt die Herstellung von Tropin aus Tropanon durch katalytische Reduktion mit durch Raney-Nickel-Katalysator angeregtem Wasserstoff ohne Angabe der Ausbeute. Die Reaktion wird unter Druck und in einem organischen Lösungsmittel, vorzugsweise Ethanol, in quantitativer Ausbeute ausgeführt.

Diese Arbeitsweise setzt einen hohen apparativen Aufwand mit Druckbehälter bzw. Autoklaven voraus. Durch den Einsatz von organischen Lösungsmitteln in Verbindung mit Raney-Nikkel-Katalysator besteht eine höhere Brand- bzw. Explosionsgefahr bei der Aufarbeitung des Reaktionsansatzes. Entsprechende Unfälle sind aus der Praxis hinlänglich bekannt.

Die US-PS 2,746,976 von 1956 beschreibt die Herstellung von Tropin im Beispiels 12 unter Verwendung eines Autoklaven als Reaktionsbehälter und Einsatz eines organischen Lösungsmittels bei einem Arbeitsdruck von 60 Atmosphären Überdruck. Auch hier entstehen die oben bereits erwähnten Nachteile.

In Chemical Abstract 99 (15), 122, 721a wird eine katalytische Hydrierung von Tropanon in Gegenwart eines Rhodium-Phosphin-Komplexes beschrieben.

Ferner sind im Stand der Technik noch weitere Verfahren beschrieben, bei denen endo-Tropin durch Hydrierung von Tropanon in Gegenwart eines Raney-Nickels-Katalysators hergestellt wird (Chemical Abstract 97 (23), 198, 428q bzw. Chemical Abstract 93 (13), 132 665a und Chemical Abstract 83 (23), 193 561d). Im erstgenannten Literaturzitat wird eine Ausbeute von 92 % angegeben.

Die bisher bekanntgewordenen Verfahren erlauben also die Herstellung von Tropin nur in Gegenwart organischer Lösungsmittel und bei Anwendung von höherem Druck.

Überraschenderweise wurde nun gefunden, daß die Möglichkeit besteht, Tropin selektiv und mit guten Ausbeuten ohne Anwendung von höherem Druck und ohne Verwendung organischer Lösungsmittel zum Tropin zu reduzieren, indem das Gemisch in wässriger Lösung bei atmosphärischem Druck und Raumtemperaturen bei 1000 bis 1500 Upm turbuliert wird. Es war nach den bisher bekanntgewordenen Verfahren nicht vorauszusehen, daß diese Reaktion glatt verläuft und darüber hinaus noch stereoselektiv die gewünschte endo-Verbindung liefert.

100 ml Raney-Nickel-Katalysator, gebrauchsfertig, d.h. mit ca. 10% Feststoffanteil, in wässriger Suspension, werden vorgelegt und darin unter Rühren 14 g Tropinon gelöst. Anschließend wird das Ganze im geschlossenen System in Wasserstoffatmosphäre drucklos bei Raumtemperatur mit ca. 1300 Umdrehungen pro Minute turbuliert, bis die Wasserstoffaufnahme beendet ist.

Danach ist das Tropinon praktisch gänzlich umgesetzt und im Gaschromatogramm lassen sich mindestens 98 Flächenprozent Tropin und nur höchstens 2 Flächenprozent Pseudotropin vermessen. Der Katalysator wird durch Filtration abgetrennt und das klare, wässrige Filtrat portionsweise mit 14 g Natriumhydroxid versetzt und anschließend mit drei Portionen á 20 ml Dichlormethan extrahiert. Die Extrakte werden vereinigt und eingeengt. Als Rückstand verbleiben 13,5 g farbloses, dickes Öl, das nach kurzem Stehen durchkristallisiert. Die weißen Kristalle schmelzen bei etwa 60°C.

## Patentansprüche

1. Verfahren zur Herstellung von endo-Tropin durch Reduktion von Tropanon mit katalytisch durch Raney-Nickel oder Raney-Kupfer erregtem Wasserstoff, dadurch gekennzeichnet, daß man das Tropanon in wässriger Lösung mit Wasserstoff bei atmosphärischem Druck und Raumtemperatur im geschlossenen System bei 1000 bis 1500 Umdrehungen pro Minute turbuliert.

2. Verfahren nach Anspruch 1, dadurch gekennzeichnet, daß bei 1300 Umdrehungen pro Minute turbuliert wird.

3. Verwendung eines schnellaufenden Rührwerkes zur Durchführung des Verfahrens nach Anspruch 1 oder 2.

4. endo-Tropin, hergestellt nach dem Verfahren der Ansprüche 1 oder 2 unter Verwendung eines schnellaufenden Rührwerkes.

## Claims

1. Process for the production of endotropine by the reduction of tropanone with hydrogen catalytically activated by Raney nickel or Raney copper, characterized in that the tropanone, in aqueous solution with hydrogen, in a closed system, at atmospheric pressure and ambient temperature is subject to turbulent action at 1000 to 1500 r.p.m.

2. Process according to claim 1, characterized in that turbulent action takes place at 1300 r.p.m.

3. Use of a high-speed stirrer for performing the process according to claim 1 or 2.

4. Endotropine, produced according to the process of claims 1 or 2, using a high-speed stirrer.

## Revendications

1. Procédé pour la préparation de l'endotropine par réduction de tropanone à l'aide d'hydrogène activé catalytiquement par du nickel de Raney ou du cuivre de Raney, caractérisé en ce que l'on fait tourbillonner la tropanone dans une solution aqueuse avec de l'hydrogène à pression atmosphérique et à température ambiante dans un système fermé à une vitesse de 1000 à 1500 tours par minute.

2. Procédé selon la revendication 1, caractérisé en ce que l'on fait tourbillonner à 1300 tours par minute.

3. Utilisation d'un agitateur à régime rapide pour réaliser le procédé selon la revendication 1 ou 2.

4. Endotropine produite selon le procédé de la revendication 1 ou 2 en utilisant un agitateur à régime rapide.
